# EUROPEAN PATENT SPECIFICATION

(11) **EP 2 094 340 B1**
(45) Date of publication and mention of the grant of the patent: **05.09.2012**
(21) Application number: 07847267.7
(22) Date of filing: 22.11.2007
(51) Int. Cl.: A61M 5/42, A61B 5/157

(54) **AN AUTOMATIC MINIATURE INJECTOR AND SAMPLE-TAKER DEVICE FOR MEDICAL USE**
AUTOMATISCHES MINIATUR-INJEKTIONSGERÄT UND PROBENEHMER FÜR MEDIZINISCHE VERWENDUNGSZWECKE
DISPOSITIF DE PRELEVEMENT D'ECHANTILLONS ET D'INJECTION MINIATURE AUTOMATIQUE A USAGE MEDICAL

(30) Priority: 24.11.2006 FR 0655091
(43) Date of publication of application: 02.09.2009
(73) Proprietor: Eveon, 38330 Montbonnot Saint Martin (FR)
(72) Inventor: PERRIERE, Bernard, 38130 ECHIROLLES (FR)
(74) Representative: Prugneau, Philippe
(86) International application number: PCT/EP2007/062676
(87) International publication number: WO 2008/062032

(56) References cited:
- WO-A-02/094093
- WO-A-2006/120619
- US-A1- 2001 041 904
- US-A1- 2003 069 509
- US-A1- 2003 167 021

## Description

The invention relates to a miniature device for medical use, suitable, in automatic manner, both for injecting a medicinal substance and for taking a (blood or other) sample in cutaneous, subcutaneous, intravenous, intramuscular, or intradermal manner with a human being or an animal.

In particular, the invention provides a device for medical use that is suitable for enabling one or more injections to be made and/or samples to be taken painlessly and without apprehension by one or more people on their own or under medical supervision.

In general, transdermal devices are all in the form of syringes or "patches". By way of example, the syringes may be syringe pens, syringe carriers, or more generally injector devices to be held in the hand by a user. Patches are extra flat systems for injecting by using micro-needles, and that are fastened to the skin by any type of means, e.g. adhesive means, Velcro strips, elastic bands, etc.

Transdermal devices in the form of syringes or patches have a needle that is visible and of dimensions that are sufficient in particular to enable the user to aim for a vein or more generally any injection target that is visible to the eye.

With certain patients, using syringes is sometimes psychologically difficult or even traumatic. Thus, merely seeing a needle can make them faint or can stress them. Some people even find it impossible to inject themselves. That can have dramatic consequences for patients who are isolated, e.g. suffering from diabetes, requiring insulin to be injected repeatedly, or in the event of seizures, occurring in unexpected or unpredictable manner.

Intradermal devices, also know as micro-needle implants, are generally in the form of micro-needle implants placed under the dermis, in a muscle, and more precisely on a vein or near a zone of cartilage. With such implants, it can also be necessary to carry out repeated injections and sample-taking operations on a given patient.

There thus exists a need for a device to inject a medicinal substance in repeated manner that can also be used to take repeated samples, e.g. of blood. In addition, it would also be advantageous to be able to alternate between taking a sample and making an injection while using the same device.

Patent document DE 42 21 312 discloses a transdermal device suitable for performing intravenous and intramuscular injections. A medicinal substance is injected using Doppler effect sensors that enable the direction and the depth of a blood vessel to be detected while the device is moving. The user must then cause a clock indicator to correspond with the direction of the vein. That manipulation is complex and difficult, and can be performed only by qualified medical personnel.

Consequently, with such a device, it is not possible automatically to perform injections of medicinal substances and to take blood samples. It is even less conceivable that patients should perform such injections or sample-taking operations on themselves.

Patent application WO 2006/120619 A discloses a system for injection and sample-taking operation which enables a needle connected to a reservoir to be inserted into the body of a patient, the system comprising particularly a detector system for the detection of a blood vessel, a control unit for actuating the needle in response to this detection. Patent application US 2003/069509 A1 discloses another system for sample-taking operation able to determine a suitable sample-taking site, comprising at least one needle which can be actuated and means to detect a blood vessel. However, these two systems do not allow for an injection and a sample-taking operation to be readily and successively performed.

The object of the present invention is to thus to provide a device that is capable of performing injections and/or sample-taking operations in automatic manner so as to facilitate the work of medical personnel, to increase the safety of patients, and to be suitable for being used by patients on themselves without fear.

To this end, the invention provides an automatic and miniaturized injector and/or sample taker device for medical are as defined by claim 1.

It will be understood that the invention thus makes it possible to perform an intravenous injection automatically. By detecting a vessel, it should also be understood that in the absence of a vessel in a predetermined volume, the sensor delivers information to the control unit. The control unit is then informed in real time that there is no vessel in the predetermined volume, thus making it possible to perform an intramuscular injection or a subcutaneous injection.

In this way, the user does not need to look at the needle and neither is it necessary for-needles to be of such a size as to enable the user to aim at a blood vessel.

In a particular embodiment of the invention, the device includes micro-electromechanical means for putting the needle into contact with an anesthetic and/or disinfectant substance by spraying.

In another particular embodiment of the invention, the device includes means suitable for pushing said substance for injection out from the reservoir in order to perform the injection, which means may be pneumatic means or a micro-electromechanical system.

In another particular embodiment of the invention, the device includes the following features:
· a micro-electromechanical system suitable for moving the needle relative to the enclosure;
· means suitable for analyzing a sample;
· means suitable for transmitting a signal representative of the analysis to an external appliance or to another injector and sample-taker device;
· signaling means providing a sound and/or visual signal suitable for providing information representative of the signal received by the detector system; · an adhesive surface;
· an independent electricity supply; and/or
· a microlaser, or a microwave or ultrasound gun for disinfecting the needle.

In another particular embodiment of the invention, the device includes means suitable for attracting into the reservoir a sample of said substance being taken, which means may be pneumatic means or a micro-electromechanical system.

In a particular embodiment of the invention, each reservoir is deformable under the action of pressure exerted by an electromechanical system.

In another particular embodiment of the invention, the device includes a plurality of reservoirs connected respectively to corresponding needles and to corresponding pump devices, each reservoir with a corresponding needle and a corresponding pump device forming a segment, said segments being grouped together in mutually integral manner to form a cartridge that is removable relative to the enclosure, and said control unit is designed to act on each segment in individual manner.

In another particular embodiment of the invention, the unit includes a rotary drive system designed to turn on the cartridge inside the enclosure so that each segment is moved successively into register with the electromechanical system.

Thus, each reservoir associated with a corresponding needle and with a corresponding pump system forms overall an independent and self-contained segment of the cartridge. It can thus be understood that with such a device, it is possible to implement in succession one or more injections and sample-taking operations in automatic manner.

A particular embodiment of the automatic injector and sample-taker device of the invention is described in greater detail below and shown in the drawings. The description is given solely by way of indicative and non-limiting example of the invention.
Figure 1 shows the device of the invention in the form of syringe in an axial section view.
Figure 2 shows the Figure 1 device in axial section view with the various separable elements spaced apart.
Figure 3 is a plan view in section of the cartridge of the device of the invention shown in Figure 1.
Figure 4 is a chart showing the operation of the Figure 1 device for an injection.
Figure 5 is a chart showing the operation of the Figure 1 device for taking a sample.
Figure 6 is a view of the device of the invention implemented a device that is particularly flat, also known as a "patch", and shown in axial section view.

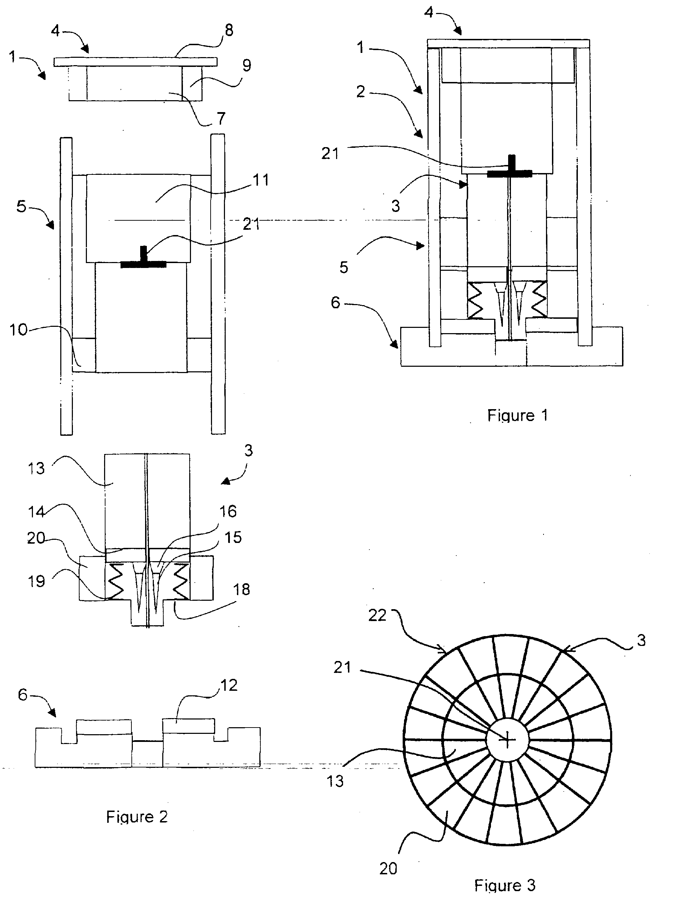

Figure 1 shows an injection and sample-taker device 1 of the invention in the particular configuration of a syringe. The device 1 is generally cylindrical in shape and comprises an enclosure 2 having a removable cartridge 3 placed therein.

As shown in Figure 2, the enclosure 2 is made up of three mutually separable elements: a top cover 4; a central cylindrical body 5; and a detector base 6. The central cylindrical body 5 has a hollow configuration for receiving the cartridge 3 via one of its ends. The other end is for receiving the top cover 4.

When the cartridge is inserted in the central cylindrical body 5, it is held by the base 6. The injection and sample-taker device 1 in the form of a syringe is thus substantially cylindrical with a top cover 4 at one end and a detector base 6 at its opposite end.

In particular, the top cover 4 is made up of a control unit 7, a display 8 placed on the outside portion of the top cover 4, and a transceiver module 9, e.g. operating at radio frequency. The display 8 may be a liquid crystal display (LCD) screen or a light-emitting diode (LED) matrix. Alternatively, the display 8 may be replaced by a system suitable for producing a sound signal.

The central body 5 mainly comprises a reader and analyzer system 10, a miniature electromechanical system 11, and a rotary drive system 21 for turning the cartridge 3 inside the enclosure 2 about the axis. The term miniature electromechanical system or micro-electrical mechanical system (MEMS) is used to designate any system fabricated by implementing the techniques that are used in the electronics industry in order to make miniature mechanisms. In this embodiment, the electromechanical system 11 is constituted, for example, by a piston together with drive means therefor.

In particular, the reader system 10 is capable of identifying the content of each reservoir 13 of the cartridge, e.g. by means of a bar code reader. Advantageously, once the cartridge 3 has been inserted in the enclosure 2, the reader system 10 enables the device 1 to determine whether the cartridge 3 contains substances for intravenous injection, for example. Advantageously, the reader system 10 also provides each cartridge with traceability.

The detector base 6 mainly comprises a detector system 12. Various types of sensor can be used in the detector system 12 depending on the type of intervention: intravenous; intramuscular; or subcutaneous. For example, it is possible to use Doppler effect sensors, infrared or pressure sensors, or indeed sensors that operate on radar principles, or optical sensors. Depending on the type of sensor used, the area and the volume probed may be prismatic, cylindrical, or conical. For example, it is possible to use infrared or ultrasound sensors.

By way of example, the detector system 12 determines the thickness of the dermis, of the fatty mass, the size and the depth of all blood vessels, the density of a muscle mass, or the presence of a zone of bone or cartilage.

Figure 3 shows the top of a removable cartridge 3 as shown in Figures 1 and 2, and made up, in accordance with the invention, of a plurality of segments 22 disposed in circular manner around the longitudinal axis of the cartridge 3. Each segment 22 is itself generally made up of a reservoir 13, of a needle, of a pump device 14 and of a disinfection and anesthesia system 20. The segments 22 are securely grouped together, e.g. by means of walls of plastics material.

In particular, each segment 22 includes a reservoir 13 of medicinal substance or for a sample of blood with a pump device 14 being fastened to the bottom portion thereof. A needle 15 is fastened to the bottom portion of the pump device 14 via a shape memory cone 16. The cone is made of a metal presenting shape memory that is capable of deforming, and in particular of expanding when an electrical or thermal excitation is applied thereto. The shape memory cone 16 can thus be calibrated to expand through a certain precise distance under a given excitation.

The needles are specific to each type of device: syringes; patches; or implants. By way of example, needle size may lie in the range 0.5 mm to 8 mm depending on the applications and the devices. The needles are made of various materials such as, for example: stainless steel; steel alloys; injected or composite plastics materials; ceramics; nanotubes; .... The needles are suitable for retaining an outer layer of substances that are disinfectant and/or anesthetic and/or healing, retention being by any type of method: such as microgrooves or vents; deposits of substances in gel or film form; pores in ceramics; or chemical combinations in nanotubes. In certain applications, these nanotube needles remain implanted and become slowly diluted in the body by chemical reactions so as to enable localized treatments to be performed without diffusing the substance throughout the human body. Certain nanotube needles present the feature of "releasing" the final portion of the tube by chemical dilution or by thermal melting so as to enable a flow, of blood or of some other fluid, to entrain said portion of the nanotube as close as possible to the zone for treatment, and once in contact with the zone or the disease for treatment, to react chemically and spread the treatment substance.

Advantageously, the removable cartridge 3 has only those elements that are needed for repeated sample-taking and/or injection on one or more patients. Consequently, the cartridge 3 is a consumable that needs to be renewed so its cost is kept as low as possible. The use of a renewable consumable avoids spending time performing the handling necessary for disassembly and sterilization.

The reservoir 13, the pump device 14, the needle 15, and the cone 16 form an integral assembly capable of moving in the enclosure 2 relative to the stationary bottom portion 18 of the cartridge 3 which bears against the base 6. The assembly comprising the reservoir 13, the pump device 14, the needle 15, and the cone 16, moves in translation along the longitudinal axis of the device 1.

A resilient element such as a spring 19 placed between the stationary bottom portion 18 of the cartridge 3 and the pump device 14 exerts a return force to maintain a gap between the pump device 14 and the stationary bottom portion 18 so that the needles 15 do not project out from the cartridge 3. Thus, patients are not traumatized, since the needle is not visible whether before injection or while injection is taking place.

Advantageously, each segment 22 of the cartridge 3 can also be provided with a disinfection and anesthesia system 20. The disinfection and anesthesia system 20 is disposed in stationary manner on the stationary bottom portion 18 around the moving assembly.

The control unit 7 is connected to the detector system 12 so as to receive the information as detected, in particular about the position and the depth of a blood vessel. The control unit 7 is also connected to the system 10 for reading and analyzing the cartridges 3, to the electromechanical system 11, to the display 8, to the transceiver module 9, to the pump device 14, to the shape memory cone 16, and to the disinfection and anesthesia system 20.

Advantageously, the above-described device comprises elements that are mutually separable and that, in association with the replaceable cartridge system, serve to provide a device that is modular and adaptable as a function of the therapies, injections, or sample-taking actions to be performed.

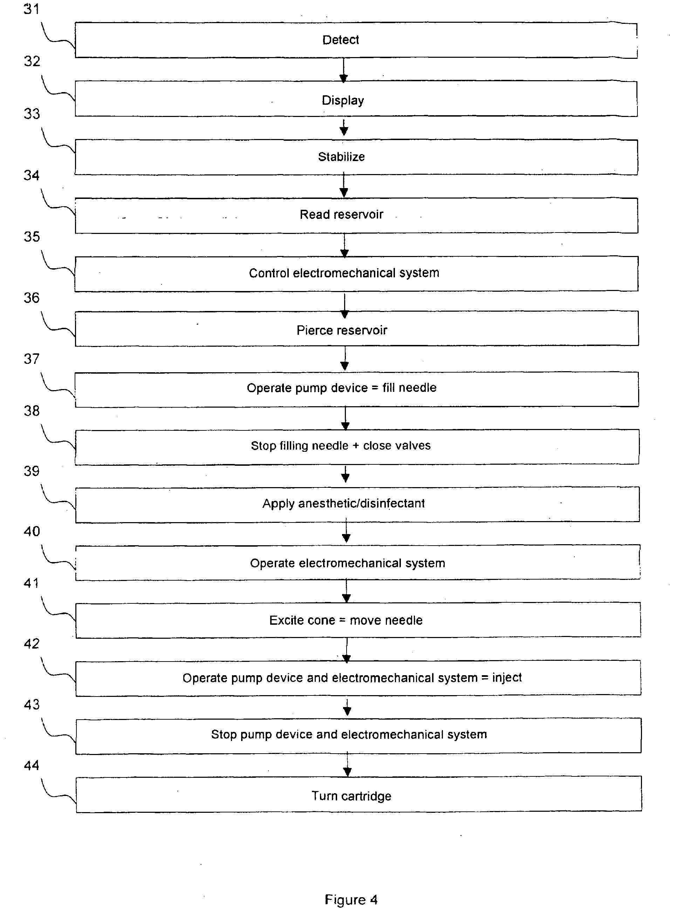

Figure 4 is a chart for explaining the operation of the syringe device 1 of the invention while performing an injection.

In a step 31, the user places the injection and sample-taker device 1 on the patient's skin with the detector base 6 in contact with the skin. The detector system 12 detects the position and the depth of the blood vessel and delivers the information to the control unit 7.

Prior reading by the reader system 10 serves to identify the substance for injection and the corresponding mode of injection, e.g. intravenous injection. Alternatively, the user may inform the device 1 that it is to perform an intravenous injection.

In a step 32, the unit 7 controls the display 8 to produce a signal informing the user that the blood vessel has been detected. Alternatively, detection of the blood vessel may be signaled to the user using a display device that is external to the device 1, e.g. a laptop computer. Under such circumstances, the information is transmitted by the transceiver module 9.

In a step 33, when a counter triggered in step 32 by the unit 7 has measured a certain lapse of time during which the injector device 1 has remained stationary, e.g. two seconds, the unit 7 causes the display 8 to produce a signal indicating that the device 1 has been stabilized over the detected blood vessel. The information indicating that the device 1 has become stabilized is produced by the detector base 6, and it corresponds to the device 1 occupying a position that is stationary relative to the blood vessel and that is suitable for making an injection.

In a step 34, the reader system 10 identifies the medicinal substance contained in the reservoir 13 that is ready for injection, and it delivers a signal to the unit 7 so that it verifies that the substance does indeed correspond to the desired injection. The substance desired for injection may have been selected beforehand by the user, e.g. using a laptop computer and acting via the transceiver module 9 and the control unit 7.

In a step 35, the unit 7 operates the electromechanical system 11 so that it exerts pressure on the reservoir 13 identified in step 34. The reservoirs 13 are made of deformable material and are provided on their top portions with rigid covers. Consequently, under the pressure exerted by the electromechanical system 11, the rigid cover moves along the inside walls of the enclosure 5.

In a step 36, the unit 7 controls a hollow rigid tube (not shown) so that it projects from the pump device 14 towards the reservoir 13 identified in step 34, thereby having the effect of piercing the reservoir 13 via a capsule provided for this purpose. The content of the reservoir 13 thus remains completely sterile.

In a step 37, the unit 7 causes the pump device 14 to entrain the medicinal substance towards the needle 15, e.g. by means of a pump (not shown). The unit 7 may also cause the electromechanical system 11 to continue to exert controlled pressure helping to deliver the substance from the reservoir 13, thus making it easier to fill the needle.

In a step 38, the pump device 14 has already measured the flow rate of the medicinal substance passing through it, so it knows when the needle 15 is full of substance, at which point it stops pumping and signals this event to the unit 7, which likewise causes the electromechanical system 11 to stop. At the end of step 38, the air contained in the pump device 14 and in the needle 15 has been expelled completely and replaced by the medicinal substance.

Valves are closed to prevent the substance contained in the needle and in the pump device from returning into the reservoir 13.

In a step 39, the unit 7 causes the disinfection and anesthesia system 20 to apply a drop of substance on the needle 15 by using pumps (not shown). By way of example, the pump may be a simple or a reversible pump or it may be a simple or double-acting diaphragm pump, or indeed it may be a pump operating by deforming blades of bimetallic strips.

The time required for pumping the disinfectant and/or anesthetic substance may be shorter or longer depending on the quantity of disinfectant and anesthetic substance that it is desired to apply. In this way, a very small quantity of substance can be applied directly to the surface of the needle, thus making it possible to reduce the quantity of substance that is used. Alternatively, the substance may be applied by spraying or by electro-ionization.

In particular, the disinfection and anesthesia system 20 may include a reservoir of substance that is perforated by a hollow rigid tube like the reservoir 13. The perforation system and the pumps are preferably provided on the detector base 6 so as to reduce the cost of the removable cartridge 3.

Naturally, steps 37 and 39 are not necessarily successive, and on the contrary they could be performed simultaneously.

In a step 40, the unit 7 causes the electromechanical system 11 to exert pressure again on the reservoir 13, with the valves remaining closed, so that the exerted pressure has the effect of pushing the integral assembly constituted by the reservoir 13, the pump device 14, the needle 15, and the cone 16 so that it moves inside the enclosure 2, as described above, with the spring 19 then being compressed.

The pump device 14 thus comes into abutment against the stationary bottom portion 18 of the cartridge 3, thereby having the effect of bringing the needle 15 up to the skin and passing through the drop of disinfectant and anesthetic substance. The electromechanical system 11 holds the integral assembly in position.

The needle 15 is thus disinfected and the substance then flows over the surface of the dermis so as to anesthetize the portion of the human or animal body where the injection is to be performed.

In a step 41, the control unit 7 excites the shape memory cone 16 so that it deforms and entrains the needle 15 in movement to the depth of the blood vessel detected in step 31.

The needle 15 penetrates through the dermis as far as the blood vessel painlessly because of the powerful anesthetic it entrains and retains along its walls as it passes through.

In a step 42, once the needle 15 has penetrated into the blood vessel, the unit 7 causes the valve to open and operates the pump of the pump device 14 so that the medicinal substance contained in the reservoir 13 is injected into the blood vessel. The unit 7 may simultaneously cause the electromechanical system 11 to operate in such a manner as to assist in injecting the medicinal substance.

The pump device 14 also has blood pressure and flow detectors that monitor and regulate the rate at which substance is injected. Consequently, the volume of medicinal substance that is injected is measured instantaneously and injection can be interrupted at any time as a function of the application.

Check valves may also be provided if there is any danger of a flow of blood tending to push the medicinal substance back into the reservoir 13.

In a step 43, the flow detectors of the pump device 14 and the reader system 10 inform the control unit 7 that the reservoir 13 is empty. The unit 7 then causes the pump of the pump device 14 to stop, and closes the valves. Furthermore, the control unit 7 remembers that the current reservoir has been used.

The control unit 7 deactivates the shape memory cone which is then no longer excited and retracts, thereby withdrawing the needle into the injector device 1. The unit 7 also causes the electromechanical system 11 to stop, thereby leading to withdrawal of the assembly comprising the reservoir 13, the pump device 14, the needle 15 and the cone 16 into its initial position that it occupied in step 31.

In a step 44, the unit 7 controls the rotary drive system 21 to turn the cartridge 3 about its axis so as to place a new segment into register with the electromechanical system 11 with a new substance for injection or with an evacuated reservoir for taking a sample.

Naturally, a variety of electromechanical systems 11 could be provided in the invention to perform a plurality of injections simultaneously.

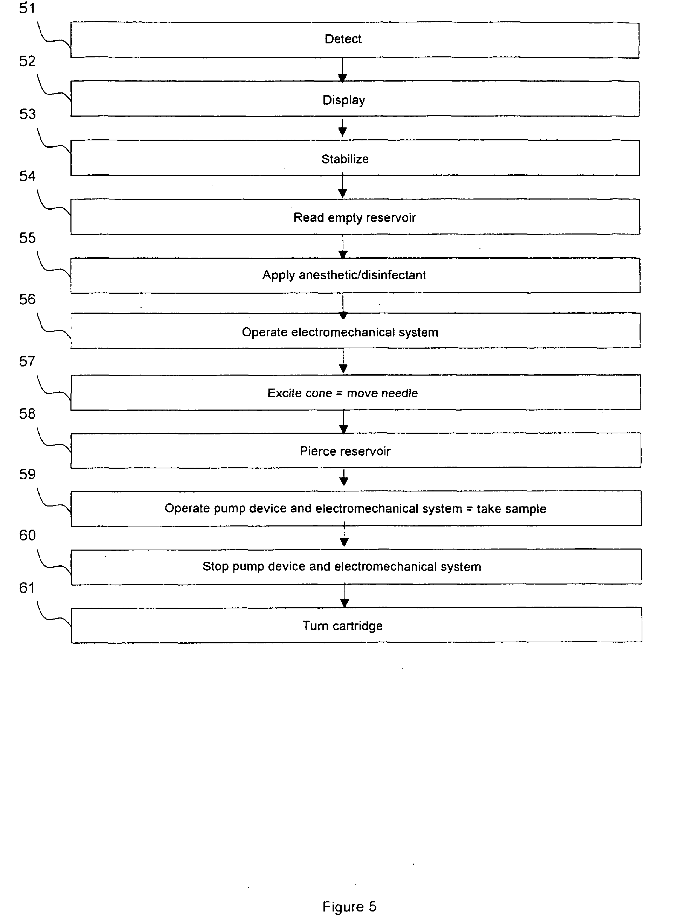

Figure 5 is a chart for explaining the operation of the syringe device 1 of the invention while taking a sample. For example, the device of the invention can be used to take a sample of blood or of other substance, to take samples of subcutaneous liquids such as synovial, lymphatic, etc.... fluids.

In a step 51, the user moves the device 1 over the skin of the patient with the detector base 6 in contact with the skin. The detector system 12 detects the position and the depth of the blood vessel and delivers that information to the control unit 7.

Prior reading by the reader system 10 has served to identify that the action to be performed is taking a blood sample and that the current reservoir 13 is empty.

In a step 52, the unit 7 causes the display 8 to produce a signal informing the user that a blood vessel has been detected.

In a step 53, when a counter triggered in step 52 by the unit 7 has measured a certain lapse of time during which the injector device 1 has remained stationary, e.g. two seconds, the unit 7 causes the display 8 to produce a signal indicating that the device 1 has become stabilized over the detected blood vessel. The information indicating that the device 1 has become stabilized is produced by the detector base 6 and corresponds to the device 1 having a stationary position relative to the blood vessel, which position is suitable for injection.

In a step 54, the reader system 10 verifies that the reservoir 13 ready for taking a sample is indeed empty.

In a step 55, the unit 7 causes the disinfection and anesthesia system 20 to apply a drop of substance on the needle 15 by using pumps (not shown).

The time required for pumping the disinfectant and/or anesthetic substance can be shorter or longer depending on the quantity of disinfectant and anesthetic substance that it is desired to apply. Alternatively, the substance may be applied by spraying or by electro-ionization.

In particular, the disinfection and anesthesia system 20 may comprise a reservoir of substance that is perforated by a hollow rigid tube like the reservoir 13. The perforation system and the pumps are preferably provided on the detector base 6 so as to reduce the cost of the removable cartridge 3.

In a step 56, the unit 7 causes the electromechanical system 11 to exert pressure on the reservoir 13 identified in step 54. Since these valves are closed, the pressure that is exerted has the effect of pushing the integral assembly constituted by the reservoir 13, the pump device 14, the needle 15, and the cone 16, which assembly moves within the enclosure 2, as described above and while compressing the spring 19.

The pump device 14 thus comes into abutment against the stationary bottom portion 18 of the cartridge 3, thereby having the effect of bringing the needle 15 up to the skin, passing through the drop of disinfectant and anesthetic substance. The electromechanical system 11 holds the integral assembly in position.

The needle 15 is thus disinfected and the substance then flows over the surface of the dermis so as to anesthetize the portion of the human or animal body where the injection is to be performed.

In a step 57, the control unit 7 excites the shape memory cone 16 which deforms and causes the needle 15 to be moved to the depth of the blood vessel detected in step 51.

The needle 15 penetrates through the dermis to the blood vessel painlessly because of the powerful anesthetic that it entrains and that remains along its walls as it passes.

In a step 58, when the needle 15 has penetrated into the blood vessel, the unit 7 causes a hollow rigid tube to project from the pump device 14 towards the reservoir 3 identified in step 54 so as to pierce the evacuated reservoir 13 via a capsule provided for this purpose. The inside of the reservoir 13 has thus remained completely sterile.

In a step 59, the unit 7 causes valves to open and the pump of the pump device 14 to operate so that blood is taken from the blood vessel and delivered into the reservoir 13. The unit 7 can also cause the_ electromechanical system 11 to act simultaneously to assist in taking the sample of blood.

The pump device 14 also has blood pressure and flow detectors that monitor and control the rate at which the sample of blood is taken. Consequently, the volume of blood that is taken is measured instantaneously and the taking of the sample can be interrupted at any moment as a function of the application.

In a step 60, since the pump device 14 has measured the rate of flow of medicinal substance passing through it, it knows that the reservoir 13 is full of blood, and therefore stops the pump of the pump device 14, closes the valves, and signals this event to the unit 7, which also causes the electromechanical system 11 to stop, thereby causing the assembly comprising the reservoir 13, the pump device 14, the needle 15, and the cone 16 to be withdrawn into the enclosure 2. Furthermore, the control unit 7 remembers that the current reservoir has been used for taking a sample.

Alternatively, the reader and analyzer system 10 can be adapted to measure the extent to which the reservoir 13 has been filled and to inform the unit 7 when the reservoir is full so that the unit 7 then stops the pump, stops the electromechanical system 11, and closes the valves.

The control unit 7 deactivates the shape memory cone which is then no longer excited and retracts, thereby moving the needle into the injector device 1. The unit 7 also stops the electromechanical system 11.

In a step 61, the unit 7 causes the rotary drive system 21 to turn the cartridge 3 about its axis so as to place a new segment in register with the electromechanical system 11 having a new substance for injection or a new evacuated reservoir for taking a sample.

Consequently, the device of the invention can be used to perform different injections of medicinal substances and to take repeated samples from a plurality of patients while using the same device having a plurality of reservoirs (segments). Furthermore, the device of the invention can also be used to take a sample and then make an injection on a single patient using a single device having a plurality of reservoirs (segments).

In a particular embodiment of the invention, one or more biochips are integrated in a reservoir 13 so as to perform one or more analyses in situ on the sample contained in the reservoir. The results of the analyses are then recorded and processed by the reader and analyzer system 10 and transmitted by the transceiver module 9 to an external appliance, e.g. a portable computer or a medical server, using wired or wireless means, with the signals that are transmitted being digital or analog, with encoding being used in certain secure applications.

When a plurality of injector and sample-taker devices 1 are used in combination both for performing a sample-taking operation and an injection operation, it is possible for the device that takes the sample to include means that are suitable for transmitting a signal representative of the analysis of a sample to another injector device. Under such circumstances, the two devices may either be provided within a common enclosure, or they may be independent, so as to make it possible to perform sample-taking and injection operations on zones that are spaced apart.

In a particular embodiment of the invention, the disinfection system 20 may be replaced by a microlaser device or a microwave or ultrasound gun. A microlaser device is suitable both for disinfecting the needle and also for disinfecting the skin without thereby burning the dermis.

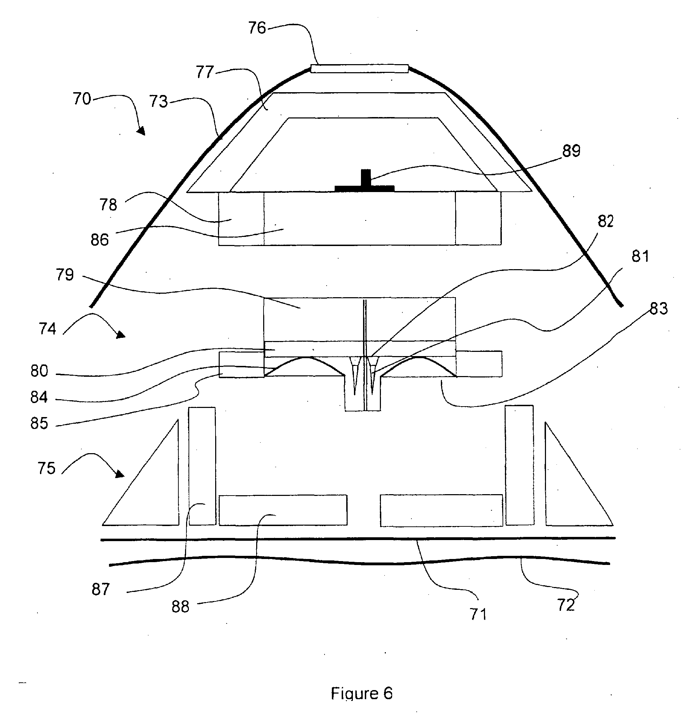

In a variant embodiment shown in Figure 6, the invention can be applied to extra flat devices for making micro-injections by means of needles, where such devices are also referred to as "patches". Under such circumstances, the injector and sample-taker device 70 has an adhesive surface 71 that is compatible with the skin 72. In this way, the device is suitable for repeatably performing sample-taking operations followed by analyses and injections without it being necessary to reposition the patch as a function of the presence or absence of a blood vessel.

Figure 6 shows the injector and sample-taker device 1 of the invention in the particular configuration of a patch that can be relatively flexible. The device 70 is generally flat in shape at its adhesive surface 71 and relatively bulging in shape over its top portion. Patch devices 70 comprise three mutually separable elements: a bulging enclosure 73; a cartridge 74 disposed in the enclosure; and a detector base 75 holding the cartridge 74 inside the enclosure 73.

In particular, the enclosure 73 has a display 76 placed on the outside portion of the enclosure, a control unit 77, a transceiver module 78, e.g. operating at radio frequency, a rotary drive system 89 for turning the cartridge 70 about the axis, and a miniature electromechanical system 86.

By way of example, the display 76 may comprise an LCD screen or an LED matrix. Alternatively, the display 76 may be replaced by a system suitable for producing a sound signal.

In the invention, the removable cartridge 74 comprises more particularly a plurality of segments distributed around the longitudinal axis of the cartridge 74. Each segment comprises a reservoir 79 of medicinal substance or for containing a blood sample, e.g. having a pump device 80 fastened to the bottom portion thereof.

A needle 81 is fastened to the bottom portion of the pump device 80 via a shape memory cone 82. The cone is made of a shape memory metal that is capable of deforming, and in particular of expanding on having electrical or -thermal excitation applied thereto. The shape memory cone 82 can thus be calibrated to expand through a precise distance under given excitation.

The reservoir 79, the pump device 80, the needle 81, and the cone 82 form an integral assembly capable of moving within the enclosure 73 relative to a stationary bottom portion 83 of the cartridge 74, which portion bears against the base 75. The assembly comprising the reservoir 79, the pump device 80, the needle 81, and the cone 82 can move in translation within the enclosure 73.

A resilient diaphragm 84 placed between the stationary bottom portion 83 of the cartridge 74 and the pump device 80 exerts a return force serving to keep a gap between the pump device 80 and the stationary bottom portion 83 so that the needles 81 do not project out from the cartridge 74. Patients are thus not traumatized, since the needle is not visible whether before injection or during injection.

Advantageously, each segment of the cartridge 74 may also be provided with a disinfection and anesthesia system 85. The disinfection and anesthesia system 85 is placed stationary on the stationary bottom portion 83 around the moving assembly.

The detection base 75 mainly comprises a system 87 for reading and analyzing the cartridges 74, and a detector system 88.

In particular, the reader system 87 is capable of identifying the content of each reservoir 79 of the cartridge, for example by means of a bar code reader. Advantageously, once the cartridge 74 has been inserted in the enclosure 73, the reader system 87 enables the device 70 to determine that the cartridge 74 contains substances for an intravenous injection, for example.

In the detector system 88, various types of sensor can be used depending on the type of injection: intravenous, intramuscular, or subcutaneous. By way of example, it is possible to use Doppler effect sensors, infrared or pressure sensors, or indeed sensors operating on radar principles or optical sensors. Depending on the type of sensor used, the surface and the volume probed may be prismatic, cylindrical, or conical. For example, it is possible to use infrared or ultrasound sensors.

The detector system 88 then detects, for example, the thickness of the dermis, the thickness of the fatty mass, the size and the depth of any blood vessels, the density of a muscle mass, or the presence of a zone of bone or of cartilage.

The control unit 77 is connected to the detector system 88 so as to receive the detected information, in particular about the position and the depth of a blood vessel. The control unit 77 is also connected to the system 87 for reading and analyzing cartridges 74, to the electromechanical system 86, to the display 76, to the transceiver module 78, to the pump device 80, to the shape memory cone 82, and to the disinfection and anesthesia system 85.

Advantageously, the above-described device comprises elements that are mutually separable that, in association with the replaceable cartridge system, serve to obtain a device that is modular and adaptable as a function of the therapies, injections, or sampling operations that are to be performed.

The operation of the patch device 70 is similar to that of the syringe device 1 as described in detail with reference to Figure 4 for injection and with reference to Figure 5 for sample-taking.

Furthermore, the injector and sample-taker device of the invention can be adapted in manners obvious to the person skilled in the art to an application in the form of a micro-needle implant. Under such circumstances, the implant is placed under the dermis, for example, in a muscle, on a vein, or towards a zone of cartilage.

In a variant embodiment of the invention, the segments of the cartridge can be disposed in linear manner rather than circular manner, so that there is no need to turn the cartridge in order to perform an injection or sample-taking operation. Under such circumstances, an electromechanical piston device can be provided over each segment.

In the embodiments described above, it should be understood that the shape memory cone could be replaced by any suitable micro-electromechanical system without thereby going beyond the scope of the present invention.

Furthermore, when a micro-electromechanical system is used, the device of the invention may include a self-contained electrical power supply. This power supply may be in the form of a rechargeable battery or a photocell, for example.

It can be seen from the above description that a device of the invention presents numerous advantages:
· it enables a person on their own to inject themselves or take a blood sample in safe manner;
· it enables different medicinal substances to be injected;
· it enables repeated samples to be taken from a plurality of patients using a single device and without changing the reservoir;
· it enables a sample to be taken and then an injection to be performed on a given patient using a single device and without changing the reservoir;
· it enables any stress associated with mere sight of a needle to be avoided;
· it makes the operations of taking a sample and making an injection practically painless;
· it makes it possible to reduce significantly the quantities of disinfectant, anesthetic, or healing substances that are used; and
· it reduces the risks of injection errors of the kind that are observed when the needle is wrongly positioned.

## Claims

1. An automatic and miniaturized injector and/or sample-taker device (1; 70) for medical use comprising an enclosure (2; 73) extending along a longitudinal axis, at least one needle (15; 81) that is movable relative to said enclosure (2; 73) along said longitudinal axis, a detector system (12; 88) suitable for probing a predetermined volume into a human or animal body to determine at least a depth information according to which the needle has to penetrate said body and a control unit (7; 77) suitable for causing the needle to move as a function of the depth information supplied by said detector system (12; 88), the device being **characterized in that** it further comprises a pump device (14; 80) for entraining a substance to be injected or a sample to be taken between the needle and a reservoir, said pump device being movably disposed in said enclosure with said needle along said longitudinal axis.

2. An injector and/or sample-taker device according to claim 1, **characterized in that** it includes means (20; 85) for putting the needle into contact with an anesthetic substance.

3. An injector and/or sample-taker device according to claim 1, **characterized in that** it includes means (20; 85) for putting the needle into contact with a disinfectant substance.

4. An injector and/or sample-taker device according to claim 1, 2 or 3, **characterized in that** it includes a microlaser, or a microwave, or ultrasound gun for disinfecting the needle.

5. An injector and/or sample-taker device according to any preceding claims, **characterized in that** said pump device is disposed between said needle and said reservoir, said reservoir with said needle and said pump device forming a segment that is removable relative to the enclosure.

6. An injector and/or sample-taker device according to claim 5, **characterized in that** it includes a plurality of reservoirs (13; 79) connected respectively to corresponding needles (15; 81) and to corresponding pump devices (14; 80), each reservoir with a corresponding needle and a corresponding pump device forming a segment (22), said segments (22) being grouped together in mutually integral manner to form a cartridge (3; 74) that is removable relative to the enclosure (2; 73), and **in that** said control unit (7; 77) is designed to act on each segment (22) in individual manner.

7. An injector and/or sample-taker device according to any preceding claims, **characterized in that** it includes means (10; 87) suitable for analyzing a sample.

8. An injector and/or sample-taker device according to claim 7, **characterized in that** it includes means (9; 78) suitable for transmitting a signal representative of the analysis to an external appliance.

9. An injector and/or sample-taker device according to claim 7, **characterized in that** it includes means (9; 78) suitable for transmitting a signal representative of the analysis to another injector and/or sample-taker device (1; 70).

10. An injector and/or sample-taker device according to claim 1, **characterized in that** it includes signaling means (8; 76) suitable for providing information representative of a signal received by the detector system (12; 88).

11. An injector and/or sample-taker device according to claim 10, **characterized in that** the signaling means (8; 76) provide a sound and/or visual signal.

12. An injector and/or sample-taker device according to any of claims 1 to 11, **characterized in that** said pump device (11, 14; 80, 86) comprises a microelectro-mechanical system.

13. An injector and/or sample-taker device according to any of claims 1 to 12, in the form of a syringe.

14. An injector and sample-taker device according to any of claims 1 to 12, in the form of a patch.

15. An injector and sample-taker device according to any of claims 1 to 12, in the form of an implant.

## Patentansprüche

1. Automatische und miniaturisierte Injektions- und/oder Probenahme-Vorrichtung (1; 70) für die medizinische Verwendung umfassend ein Gehäuse (2; 73), das sich entlang einer Längsachse erstreckt, mindestens eine Nadel (15; 81), die relativ zu dem Gehäuse (2; 73) entlang der Längsachse bewegbar ist, ein Erkennungssystem (12; 88), das geeignet ist zum Sondieren eines vorbestimmten Volumens von einem menschlichen oder tierischen Körper, um mindestens eine Tiefeninformation, wonach die Nadel in den Körper einzudringen hat, zu bestimmen und eine Steuereinheit (7; 77), die geeignet ist, die Nadel sich in Funktion der von dem Erkennungssystem (12; 88) zur Verfügung gestellten Tiefeninformation zu bewegen, wobei die Vorrichtung **dadurch gekennzeichnet ist, dass** diese weiterhin eine Pumpvorrichtung (14; 80) zum Mitnehmen einer zu injizierenden Substanz oder einer zwischen der Nadel und einem Behälter zu entnehmenden Probe umfasst, wobei die Pumpe entlang der Längsachse in dem Gehäuse mit der Nadel bewegbar angeordnet ist.

2. Injektions- und/oder Probenahme-Vorrichtung nach Anspruch 1, **dadurch gekennzeichnet, dass** diese Mittel (20; 85) zum Bringen der Nadel in Kontakt mit einer betäubenden Substanz beinhaltet.

3. Injektions- und/oder Probenahme-Vorrichtung nach Anspruch 1, **dadurch gekennzeichnet, dass** diese Mittel (20; 85) zum Bringen der Nadel in Kontakt mit einer desinfizierenden Substanz beinhaltet.

4. Injektions- und/oder Probenahme-Vorrichtung nach Anspruch 1, 2 oder 3, **dadurch gekennzeichnet, dass** diese eine Mikrolaser- oder eine Mikrowellen- oder Ultraschall-Kanone zum Desinfizieren der Nadel beinhaltet.

5. Injektions- und/oder Probenahme-Vorrichtung nach einem der vorhergehenden Ansprüche, **dadurch gekennzeichnet, dass** die Pumpvorrichtung zwischen der Nadel und dem Behälter angeordnet ist, wobei der Behälter mit der Nadel und die Pumpvorrichtung einen Abschnitt bilden, der in Bezug auf das Gehäuse entfernbar ist.

6. Injektions- und/oder Probenahme-Vorrichtung nach Anspruch 5, **dadurch gekennzeichnet, dass** diese eine Vielzahl von Behältern (13; 79) beinhaltet, die mit entsprechenden Nadeln (15; 81) und mit entsprechenden Pumpvorrichtungen (14; 80) verbunden sind, jeder Behälter mit einer entsprechenden Nadel und einer entsprechenden Pumpvorrichtung einen Abschnitt (22) bildet, wobei die Abschnitte (22) zusammen in einer gemeinsam integralen Weise gruppiert sind, um einen Einsatz (3; 74) zu bilden, der in Bezug auf das Gehäuse (2; 73) entfernbar ist, und dass die Steuereinheit (7; 77) ausgebildet ist, auf jedes Segment (22) in individueller Weise einzuwirken.

7. Injektions- und/oder Probenahme-Vorrichtung nach einem vorhergehenden Anspruch, **dadurch gekennzeichnet, dass** diese Mittel (10; 87) beinhaltet, die zum Analysieren der Probe geeignet sind.

8. Injektions- und/oder Probenahme-Vorrichtung nach Anspruch 7, **dadurch gekennzeichnet, dass** diese Mittel (9; 78) beinhaltet, die geeignet sind zum Übertragen eines für die Analyse charakteristischen Signals an ein externes Gerät.

9. Injektions- und/oder Probenahme-Vorrichtung nach Anspruch 7, **dadurch gekennzeichnet, dass** diese Mittel (9; 78) beinhaltet, die geeignet sind zum Übertragen eines für die Analyse charakteristischen Signals zu einer anderen Injektions- und/oder Probenahme-Vorrichtung (1; 70).

10. Injektions- und/oder Probenahme-Vorrichtung nach Anspruch 1, **dadurch gekennzeichnet, dass** diese Signalmittel (8; 76) beinhaltet, die geeignet sind zum Verfügungstellen von Informationen, die charakteristisch für ein von dem Erkennungssystem (12; 88) empfangenes Signal sind.

11. Injektions- und/oder Probenahme-Vorrichtung nach Anspruch 10, **dadurch gekennzeichnet, dass** die Signalmittel (8; 76) ein Tonsignal und/oder ein optisches Signal zur Verfügung stellen.

12. Injektions- und/oder Probenahme-Vorrichtung nach einem der Ansprüche 1 bis 11, **dadurch gekennzeichnet, dass** die Pumpvorrichtung (11, 14; 80, 86) ein mikroelektromechanisches System umfasst.

13. Injektions- und/oder Probenahme-Vorrichtung nach einem der Ansprüche 1 bis 12 in der Form einer Spritze.

14. Injektions- und Probenahme-Vorrichtung nach einem der Ansprüche 1 bis 12 in der Form eines Pflasters.

15. Injektions- und Probenahme-Vorrichtung nach einem der Ansprüche 1 bis 12 in der Form eines Implantates.

## Revendications

1. Dispositif d'injection et/ou de prélèvement (1;70) miniaturisé et automatique à usage médical comportant une enceinte (2;73) s'étendant selon un axe longitudinal, au moins une aiguille (15;81) mobile par rapport à ladite enceinte (2;73) le long dudit axe longitudinal, un système de détection (12;88) apte à sonder un volume prédéterminé à l'intérieur d'un corps humain ou animal pour déterminer au moins une information de profondeur selon laquelle l'aiguille a à pénétrer ledit corps et une unité de commande (7;77) apte à commander le déplacement de l'aiguille en fonction des informations de profondeur fournies par ledit système de détection (12;88), le dispositif étant **caractérisé en ce qu'**il comporte en outre un dispositif de pompage (14, 80) pour entraîner une substance à injecter ou un échantillon à prélever entre l'aiguille et un réservoir, ledit dispositif de pompage étant disposé de façon mobile dans ladite enceinte avec ladite aiguille le long dudit axe longitudinal.

2. Dispositif d'injection et/ou de prélèvement selon la revendication 1, **caractérisé en ce qu'**il comporte des moyens (20; 85) pour mettre en contact l'aiguille avec une substance anesthésiante.

3. Dispositif d'injection et/ou de prélèvement selon la revendication 1, **caractérisé en ce qu'**il comporte des moyens (20; 85) pour mettre en contact l'aiguille avec une substance désinfectante.

4. Dispositif d'injection et/ou de prélèvement selon la revendication 1, 2 ou 3, **caractérisé en ce qu'**il comporte un micro laser, un canon à micro ondes ou à ultrasons pour la désinfection de l'aiguille.

5. Dispositif d'injection et/ou de prélèvement selon l'une des revendications précédentes, **caractérisé en ce que** ledit dispositif de pompage est disposé entre ladite aiguille et ledit réservoir, ledit réservoir avec ladite aiguille et ladite pompe formant un segment amovible par rapport à ladite enceinte.

6. Dispositif d'injection et/ou de prélèvement selon la revendication 5, **caractérisé en ce qu'**il comporte une pluralité de réservoirs (13;79) connectés respectivement à des aiguilles (15;81) correspondantes et à des dispositifs de pompage (14;80) correspondants, chaque réservoir avec une aiguille correspondante et un dispositif de pompage correspondant formant un segment (22), lesdits segments (22) étant regroupés d'un seul tenant entre eux pour former une cartouche (3;74) amovible par rapport à ladite enceinte (2;73), et **en ce que** ladite unité de commande (7;77) est conçue pour agir sur chaque segment (22) de manière individuelle.

7. Dispositif d'injection et/ou de prélèvement selon l'une des revendications précédentes, **caractérisé en ce qu'**il comporte des moyens (10;87) aptes à réaliser l'analyse d'un prélèvement.

8. Dispositif d'injection et/ou de prélèvement selon la revendication 7, **caractérisé en ce qu'**il comporte des moyens (9;78) aptes à transmettre un signal représentatif de l'analyse à destination d'un appareil extérieur.

9. Dispositif d'injection et/ou de prélèvement selon la revendication 7, **caractérisé en ce qu'**il comporte des moyens (9;78) aptes à transmettre un signal représentatif de l'analyse à un autre dispositif d'injection et de prélèvement (1 ;70).

10. Dispositif d'injection et/ou de prélèvement selon la revendication 1, **caractérisé en ce qu'**il comporte des moyens de signalisation (8;76) aptes à fournir une information représentative d'un signal reçu par le système de détection (12;88).

11. Dispositif d'injection et/ou de prélèvement selon la revendication 10, **caractérisé en ce que** les moyens de signalisation (8;76) fournissent un signal sonore et/ou visuel.

12. Dispositif d'injection et/ou de prélèvement selon l'une quelconque des revendications 1 à 11, **caractérisé en ce que** ledit dispositif de pompage (11, 14; 80, 86) comporte un système micro électromécanique.

13. Dispositif d'injection et/ou de prélèvement selon l'une quelconque des revendications 1 à 12, sous la forme d'une seringue.

14. Dispositif d'injection et/ou de prélèvement selon l'une quelconque des revendications 1 à 12, sous la forme d'un patch.

15. Dispositif d'injection et/ou de prélèvement selon l'une quelconque des revendications 1 à 12, sous la forme d'un implant..
